# EUROPÄISCHE PATENTANMELDUNG

(11) **EP 1 064 997 A2**
(43) Veröffentlichungstag der Anmeldung: **03.01.2001**
(21) Anmeldenummer: 00111876.9
(22) Anmeldetag: 09.06.2000
(51) Int. Cl.: B01J 23/54, C07C 27/10

(54) **Verfahren und Katalysator zur Herstellung von C2-Oxygenaten aus Synthesegas**

(30) Priorität: 25.06.1999 DE 19929281
(71) Anmelder: BASF AKTIENGESELLSCHAFT, 67056 Ludwigshafen (DE)
(72) Erfinder: Luo, Hongyuan, Prof. Dr., Dalian 116023 (CN); Zhou, Huanwen, Dr., Dalian 116023 (CN)
(74) Vertreter: Isenbruck, Günter, Dr.

(57) **Zusammenfassung**

In dem Verfahren zur Herstellung von C₂-Oxygenaten durch Umsetzung von CO und H₂ an einem rhodiumhaltigen Trägerkatalysator enthält der Katalysator, bezogen auf das Gesamtgewicht,
- 0,01 bis 10 Gew.-%: Rhodium
- 0,001 bis 10 Gew.-%: Zirkonium
- 0,01 bis 5 Gew.-%: Iridium
- 0,01 bis 10 Gew.-%: mindestens eines Metalls, ausgewählt aus Kupfer, Kobalt, Nickel, Mangan, Eisen, Ruthenium und Molybdän
- 0,01 bis 10 Gew.-%: mindestens eines Alkali- oder Erdalkalimetalls, ausgewählt aus Lithium, Natrium, Kalium, Rubidium, Magnesium und Calcium
auf einem inerten Träger.

## Beschreibung

Die Erfindung betrifft ein Verfahren und einen Katalysator zur Herstellung von C₂-Oxygenaten aus Synthesegas, d.h. CO/H₂-Gasgemischen.

Die heterogen katalysierte Umsetzung von Synthesegas zu Kohlenwasserstoffen (Fischer-Tropsch-Synthese) beziehungsweise zu Methanol ist seit den 20er Jahren bekannt. Dagegen wurde erst 1975 entdeckt, daß an Rhodium-Katalysatoren Synthesegas direkt in C₂-Oxygenate umgewandelt werden kann.

Prinzipiell kann die direkte Umsetzung von Synthesegas an Rhodium-Katalysatoren zu einem breiten Spektrum von Produkten fuhren. So werden an nicht-promotierten Rh/SiO₂-Katalysatoren neben Methan und höheren Kohlenwasserstoffen auch Oxygenate wie Methanol, Ethanol, höhere Alkohole, Diole, Acetaldehyd und Essigsäure gebildet. Sowohl die Selektivität als auch die Aktivität von Rhodium-Katalysatoren lassen sich durch Dotierung mit anderen Elementen beziehungsweise durch entsprechende Wahl des Trägers erheblich modifizieren. Beispielsweise bewirken Dotierungen mit Lithium oder Kalium eine Unterdrückung der Methanbildung, Dotierungen mit Mangan eine beträchtliche Aktivitätssteigerung, ohne dabei die Selektivität wesentlich zu beeinflussen, und die Verwendung von CeO₂ statt SiO₂ als Träger eine wesentliche Erhöhung der Selektivität der Ethanolbildung. Darüber hinaus hängt die Produktverteilung von Reaktionsparametern wie beispielsweise Druck, Temperatur, Umsatz und CO/H₂-Verhältnis ab.

In der EP-A-0 010 295 ist ein Verfahren zur Herstellung von Ethanol aus Synthesegas beschrieben, wobei die Umsetzung an einem Rhodium-Träger-Katalysator durchgeführt wird, der als Co-Katalysator eines oder mehrere der Elemente Zirkon, Hafnium, Lanthan, Platin, Chrom und Quecksilber enthält.

EP-A-0 079 132 betrifft ein Verfahren zur Herstellung von oxygenierten Kohlenwasserstoffen durch katalytische Umsetzung von Synthesegas an einem Trägerkatalysator, der als Aktivkomponenten Rhodium, Silber, Zirkonium und Molybdän neben wahlweise zusätzlich Eisen, Mangan, Rhenium, Wolfram, Ruthenium, Chrom, Thorium und Kalium enthält. Das bevorzugte Trägermaterial ist Siliciumdioxid.

J5 9078-130-A betrifft ein Verfahren zur Herstellung von Essigsäure, Acetaldehyd und Ethanol durch Umsetzung von Synthesegas an einem Rhodiumkatalysator, der als Promotoren Mangan, Zirkonium und mindestens ein Alkalimetall enthält.

Intensiv beforscht wurde die direkte heterogenkatalysierte Gasphasensynthese von Essigsäure aus Synthesegas an Rhodium-Katalysatoren vor allem in den Jahren 1980 bis 1986 im Rahmen des japanischen C₁-Projektes 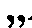 Research and Development Program for New Technologies to Produce Basic Industrial Chemicals from Carbon Monoxide and other Chemicals". Im Laufe dieser Arbeiten zur Essigsäure-Synthese wurde der Einfluß von ca 60 Elementen als Promotoren auf die Aktivität und Selektivität von heterogenen Rhodium-Katalysatoren systematisch untersucht. Es wurde hierbei festgestellt, daß Promotoren wie zum Beispiel Mg, Sc, Ti, V, Cr, Mn, Mo oder La die katalytische Aktivität erhöhen, während Promotoren wie zum Beispiel Li, K, Zr oder Ir zu einer Erhöhung der Selektivität der Essigsäurebildung führen. Am Ende des Projektes konnte mit einem optimierten Rh-Mn-Ir-Li-K/SiO₂-Katalysator eine Essigsäure-Selektivität von 71 % bei einer Raum-Zeit-Ausbeute von 344 g/l*h erreicht werden. Es wurde auch ein weiterer, nicht genauer beschriebener Katalysator entwickelt, der eine Gesamtselektivität für Essigsäure + Acetaldehyd von 90 % bei einer Raum-Zeit-Ausbeute von 480 g/l*h ergab.

Die Ergebnisse des japanischen C₁-Projekts sind in dem Buch 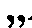 Progress in C₁ Chemistry in Japan", Elsevier, 1989 zusammengestellt, wobei speziell die Arbeiten zur Herstellung von Essigsäure aus Synthesegas in Kapitel 6 (Seiten 287 bis 330) beschrieben werden. Bei den Screening-Tests zur Katalysatoroptimierung wurde nicht nur der Einfluß von verschiedenen Promotoren auf die Katalysator-Performance aufgeklärt, sondern auch eine weitere Anzahl von Parametern untersucht, wie zum Beispiel die Testbedingungen (P, T, SV, CO/H₂-Verhältnis), eine partielle Vergiftung mit Schwefel, die Träger-Zusammensetzung und
- Porenstruktur, die Rh-Teilchengröße und die Bedingungen bei der Trocknung und bei der Reduktion des Katalysators. Ferner wurden die Reaktionskinetik an einem Rh-Mn-Ir-Li-K/SiO₂-Katalysator und das Alterungsverhalten eines Rh-Mn-Ir-Li/SiO₂-Katalysators untersucht.

Aufgabe der vorliegenden Erfindung ist die Bereitstellung eines Verfahrens und eines Katalysators zur Herstellung von C₂-Oxygenaten, der die gewünschten Produkte mit hohen Umsätzen und hohen Selektivitäten bei großer Katalysatoraktivität und Standzeit liefert.

Die Aufgabe wird erfindungsgemäß gelöst durch ein Verfahren zur Herstellung von C₂-Oxygenaten durch Umsetzung von CO und H₂ an einem rhodiumhaltigen Trägerkatalysator, bei dem der Katalysator, bezogen auf das Gesamtgewicht,
- 0,01 bis 10 Gew.-%: Rhodium
- 0,001 bis 10 Gew.-%: Zirkonium
- 0,01 bis 5 Gew.-%: Iridium
- 0.01 bis 10 Gew.-%: mindestens eines Metalls, ausgewählt aus Kupfer, Kobalt, Nickel, Mangan, Eisen, Ruthenium und Molybdän
- 0,01 bis 10 Gew.-%: mindestens eines Alkali- oder Erdalkalimetalls. ausgewählt aus Lithium, Natrium, Kalium, Rubidium, Magnesium und Calcium
auf einem inerten Träger enthält.

Es wurde erfindungsgemäß gefunden, daß der obige Katalysator bei der Umsetzung von Synthesegas zu C₂-Oxygenaten ein sehr gutes Leistungsspektrum zeigt. Der Katalysator zeigt eine hohe Aktivität und Selektivität, auch wenn der Gehalt an Rhodium sehr gering ist.

Der Katalysatorträger ist vorzugsweise ausgewählt aus SiO₂, Al₂O₃, TiO₂, Zeolithen, Aktivkohle, Diatomeenerde und Gemischen davon.

Das mindestens eine Alkali- oder Erdalkalimetall ist vorzugsweise ausgewählt aus Lithium. Kalium und Magnesium.

Das mindestens eine Metall ist vorzugsweise ausgewählt aus Kupfer, Mangan, Eisen, Ruthenium und Molybdän.

Ein bevorzugter Katalysator enthält
0,1 bis 5 Gew.-%, insbesondere 0,5 bis 3,5 Gew.-% Rhodium
0,05 bis 5 Gew.-%, insbesondere 0,08 bis 4,5 Gew.-% Zirkonium
0,1 bis 3,5, insbesondere 0,2 bis 2,5 Gew.-% Iridium
0,1 bis 5 Gew.-%, insbesondere 0,2 bis 1,5 Gew.-% mindestens eines Metalls, ausgewählt aus Kupfer, Kobalt, Nickel, Mangan, Eisen, Ruthenium und Molybdän
0,05 bis 5 Gew.-%, insbesondere 0,1 bis 1,2 Gew.-% mindestens eines Alkali- oder Erdalkalimetalls, ausgewählt aus Lithium, Natrium, Kalium, Rubidium, Magnesium und Calcium
auf einem inerten Träger.

Der erfindungsgemäße Katalysator kann dadurch erhalten werden, daß man den inerten Träger mit in wäßrigen oder organischen Lösungsmitteln gelösten Katalysatormetallverbindungen, insbesondere Katalysatormetallsalzen, imprägniert, den imprägnierten Träger trocknet und calciniert und nachfolgend reduziert. Das Imprägnieren kann dabei auf beliebige Weise erfolgen, beispielsweise durch Tränken oder Besprühen. Bevorzugt werden Katalysatormetallsalze eingesetzt, die in Wasser oder organischen Lösungsmitteln wie Ethanol löslich sind. Dabei ist die Reihenfolge, in der mit den Katalysatormetallverbindungen getränkt wird, frei wählbar. Die Tränkung kann nacheinander oder gleichzeitig mit allen Komponenten erfolgen.

Der Katalysator wird erfindungsgemäß bei der Umsetzung von Synthesegas, insbesondere zu C₂-Oxygenaten wie Ethanol, Acetaldehyd und Essigsäure eingesetzt.

Dabei wird die Umsetzung vorzugsweise bei einem Druck im Bereich von 1 bis 100 bar und bei einer Temperatur im Bereich von 200 bis 400 °C durchgeführt.

Vorzugsweise beträgt das molare Verhältnis von H₂ zu CO 10 bis 0,05 zu 1.

Die Erfindung wird nachstehend anhand von Beispielen näher erläutert.

### Beispiel 1

RhCl₃, ZrO(NO₃)₂, H₂IrCL₆, Cu(NO₃)₂, KNO₃ wurde in Wasser gelöst entsprechend des gewünschten Konzentrationsverhältnisses auf dem Träger. Sodann wurden der Träger (SiO₂ 20 bis 40 µm) für 1 bis 2 Stunden in die Lösung getaucht. Nach dem Abtropfen aus der Lösung wurde für 24 Stunden bei 50 bis 60°C getrocknet und anschließend für 6 Stunden bei 110°C calciniert. Der erhaltene Katalysator wies 1,5 Gew.-% Rh, 0,1 Gew.-% Zr, 0,3 Gew.-% Ir, 0,8 Gew.-% Cu, 0,2 Gew.-% K auf.

Vor der Umsetzung mit Synthesegas wurde der Katalysator für 3 Stunden bei 300°C mit Wasserstoff reduziert. Sodann wurde mit Synthesegas bei einer Temperatur von 300 °C umgesetzt. Der Gesamtdruck von CO und H₂ betrug 3,0 MPa, das Molverhältnis H₂/CO war 2. Die Raumgeschwindigkeit betrug 15000 h⁻¹.

Die CO-Umwandlung betrug 4,5 % die Raum-Zeit-Ausbeute an C₂-Oxygenaten 312,6 g/kg h. Die Selektivität war 70,1 % CO.

### Beispiel 2

Es wurde der gleiche Katalysator wie in Beispiel 1 eingesetzt, jedoch wurde bei der Herstellung der Träger zunächst in ZrO(NO₃)₂-Lösung getaucht, anschließend getrocknet und dann in eine Lösung getaucht, die RhCl₃, H₂IrCl₆, Cu(NO₃)₂ und KNO₃ enthielt. Anschließend wurde getrocknet.

Der CO-Umsatz betrug 4,3 %, die Raum-Zeit-Ausbeute an C₂-Oxygenaten betrug 351,2 g/kg h. Die Selektivität war 73,5 % CO.

### Beispiele 3 bis 11

Die Umsetzung erfolgte wie in Beispiel 1 beschrieben, jedoch wurde der in der nachstehenden Tabelle angegebene Katalysator eingesetzt.

Die Ergebnisse sind in der nachfolgenden Tabelle aufgeführt.

| **Beispiel** | **Katalysator** | **CO Umsatz %** | **Raum-Zeit-Ausbeute an C**_{**2**}**-Oxygenaten** | **C**_{**2**}**-oxy-Selektivität % (C)** |
|---|---|---|---|---|
| 3 | 3,5 Rh-0,2Zr-0,2Ir-0,7Cu-0,1Li/SiO₂ | 8,7 | 726,8 | 75,8 |
| 4 | 1Rh-0,08Zr-0,5Ir-0,5Mn-0,8K-0,1Li/SiO₂ | 3,4 | 273,5 | 69,2 |
| 5 | 1,5Rh-0,1Zr-0,5Ir-0,2Fe-0,1Ru-0,2K/SiO₂ | 4,8 | 414,9 | 77,8 |
| 6 | 2,0Rh-0,1Zr-1,0Ir-0,2Ru-0,5Li/SiO₂ | 5,4 | 440,2 | 73,2 |
| 7 | 0,5Rh-0,3Zr-0,8Ir-0,9Ru-0,3Mo-0,2Mg/ZSM-5 | 2,1 | 148,6 | 63,7 |
| 8 | 1,0Rh-2,8Zr-0,2Ir-1,0Mn-0,5Mo-1,2Li/SiO₂ | 4,2 | 336,5 | 72,1 |
| 9 | 1,0Rh-4,5Zr-0,5Ir-0,5Mn-0,5Cu-0,8K/SiO₂ | 3,9 | 340,2 | 78,5 |
| 10 | 1,0Rh-0,3Zr-2,5Ir-0,5Mn-0,2K/SiO₂ | 4,2 | 363,5 | 77,9 |
| 11 | 1,0Rh-0,3Zr-2,5Ir-0,5Mn-0,2K/Al₂O₃ | 5,9 | 362,1 | 55,7 |
| V1 | 1,0Rh-0,5Mn-0,2K/SiO₂ | 1,2 | 96,7 | 65,3 |
| V2 | 1,0Rh-2,5Ir-5Mn-2K/SiO₂ | 3,9 | 289,4 | 66,8 |

## Patentansprüche

1. Verfahren zur Herstellung von C₂-Oxygenaten durch Umsetzung von CO und H₂ an einem rhodiumhaltigen Trägerkatalysator, dadurch gekennzeichnet, daß der Katalysator, bezogen auf das Gesamtgewicht,
0,01 bis 10 Gew.-% Rhodium
0,001 bis 10 Gew.-% Zirkonium
0,01 bis 5 Gew.-% Iridium
0,01 bis 10 Gew.-% mindestens eines Metalls, ausgewählt aus Kupfer, Kobalt, Nickel, Mangan, Eisen, Ruthenium und Molybdän
0,01 bis 10 Gew.-% mindestens eines Alkali- oder Erdalkalimetalls, ausgewählt aus Lithium, Natrium, Kalium, Rubidium, Magnesium und Calcium
auf einem inerten Träger enthält.

2. Verfahren nach Anspruch 1, dadurch gekennzeichnet, daß der Katalysatorträger ausgewählt ist aus SiO₂, Al₂O₃, TiO₂, Zeolithen, Aktivkohle und Diatomeenerde.

3. Verfahren nach Anspruch 1 oder 2, dadurch gekennzeichnet, daß das mindestens eine Alkali- oder Erdalkalimetall ausgewählt ist aus Lithium, Kalium und Magnesium.

4. Verfahren nach einem der Ansprüche 1 bis 3, dadurch gekennzeichnet, daß das mindestens eine Metall ausgewählt ist aus Kupfer, Mangan, Eisen, Ruthenium und Molybdän.

5. Verfahren nach einem der Ansprüche 1 bis 4, dadurch gekennzeichnet, daß die Umsetzung bei einem Druck im Bereich von 1 bis 100 bar und bei einer Temperatur im Bereich von 200 bis 400°C durchgeführt wird.

6. Verfahren nach einem der Ansprüche 1 bis 5, dadurch gekennzeichnet, daß das molare Verhältnis von H₂ zu CO 10 bis 0,05 zu 1 beträgt.

7. Rhodiumhaltiger Trägerkatalysator, enthaltend, bezogen auf das Gesamtgewicht,
0,01 bis 10 Gew.-% Rhodium
0,001 bis 10 Gew.-% Zirkonium
0,01 bis 5 Gew.-% Iridium
0,01 bis 10 Gew.-% mindestens eines Metalls, ausgewählt aus Kupfer, Kobalt, Nickel, Mangan, Eisen, Ruthenium und Molybdän
0,01 bis 10 Gew.-% mindestens eines Alkali- oder Erdalkalimetalls, ausgewählt aus Lithium, Natrium, Kalium, Rubidium, Magnesium und Calcium
auf einem inerten Träger.

8. Verfahren zur Herstellung eines Katalysators gemäß Anspruch 7, dadurch gekennzeichnet, daß man den inerten Träger mit in wäßrigen oder organischen Lösungsmitteln gelösten Katalysatormetallverbindungen imprägniert, den imprägnierten Träger trocknet und calciniert und nachfolgend reduziert.

9. Verwendung eines Katalysators nach Anspruch 7 bei der Umsetzung von Synthesegas.
